# EUROPÄISCHE PATENTSCHRIFT

(11) **EP 0 903 434 B1**
(45) Veröffentlichungstag und Bekanntmachung des Hinweises auf die Patenterteilung: **27.11.2002**
(21) Anmeldenummer: 98810890.8
(22) Anmeldetag: 08.09.1998
(51) Int. Cl.: D06M 13/358, C07D 251/28, C07D 251/44, D06P 1/642

(54) **Verfahren zur Behandlung von Cellulosefasern**
Process for the treatment of cellulosic fibres
Procédé pour le traitement de fibres cellulosiques

(30) Priorität: 17.09.1997 EP 97810671; 25.03.1998 CH 70598
(43) Veröffentlichungstag der Anmeldung: 24.03.1999
(73) Patentinhaber: LENZING AKTIENGESELLSCHAFT, 4860 Lenzing (AT)
(72) Erfinder: Aeschlimann, Peter, 4123 Allschwil (CH)
(74) Vertreter: Schwarz, Albin, Dr.

(56) Entgegenhaltungen:
- EP-A- 0 538 977
- EP-A- 0 616 071
- WO-A-97/49856
- DE-A- 1 964 619
- DE-B- 1 085 492
- DE-B- 1 141 612
- DE-B- 1 141 973
- DE-B- 1 148 222
- FR-A- 1 243 816
- GB-A- 896 814
- DATABASE WPI Section Ch, Week 8111 Derwent Publications Ltd., London, GB; Class B02, AN 81-18088D XP002091215 & JP 56 002975 A (SUMITOMO CHEM CO LTD) , 13. Januar 1981

## Beschreibung

Die vorliegende Erfindung betrifft ein Verfahren zur Verringerung der Fibrillierungsneigung bei Lyocell-Cellulosefasern.

Als Lyocell werden solche Fasern bezeichnet, die durch ein Verfahren erhalten werden, bei dem die Cellulose in einem organischen Lösungsmittel, einer Kombination eines organischen Lösungsmittels mit einem anorganischen Salz oder in wässrigen Salzlösungen gelöst, und anschliessend aus dieser Lösung gesponnen wird.

Die Brauchbarkeit von aus den genannten Fasern hergestellten Flächengebilden, z.B. Textilmaterialien, wird jedoch durch die ausgeprägte Neigung dieser Fasern, im nassen Zustand zu fibrillieren, stark eingeschränkt. Unter Fibrillierung wird das Aufbrechen der nassen Faser in Längsrichtung bei mechanischer Beanspruchung bis zum Ablösen von Fibrillen entlang der Faseroberfläche verstanden, wodurch die Faser ein haariges oder pelziges Aussehens erhält. Ein aus diesen Fasern hergestelltes Gewebe verliert ausserdem nach einigen Wäschen stark an Farbintensität.

Es besteht somit ein Bedarf nach einem Verfahren, das die Fibrillierung bei Lyocell-Fasern verringert oder diese vollständig unterdrückt.

Es hat sich überaschenderweise gezeigt, dass durch das erfindungsgemässe Verfahren die Fibrillierungsneigung der behandelten Lyocell-Fasern stark verringert wird.

Gegenstand der vorliegenden Erfindung ist somit ein Verfahren zur Behandlung von Lyocell-Cellulosefasern, dadurch gekennzeichnet, dass man die Lyocell-Cellulosefaser mit mindestens einer Verbindung der Formel 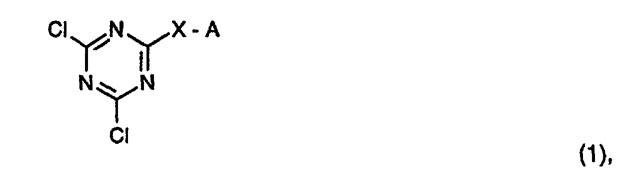 worin
X -NR- oder -S- ist,
A einen aliphatischer Rest mit 1 bis 18 C-Atomen, der frei von Sulfogruppen ist, bedeutet, oder -X-A Hydroxy ist, und
R Wasserstoff ist oder die Bedeutung von A unabhängig von A hat,
behandelt.

Üblicherweise behandelt man die Lyocell-Cellulosefaser mit mindestens einer Verbindung der Formel (1), worin X -NR- oder -S- ist, A einen aliphatischer Rest mit 1 bis 18 C-Atomen, der frei von Sulfogruppen ist, bedeutet, und R Wasserstoff ist oder die Bedeutung von A unabhängig von A hat.

A als aliphatischer Rest mit 1 bis 18 C-Atomen ist z.B ein C₁-C₁₈-Alkylrest, wie z.B. Methyl, Ethyl, n-Propyl, iso-Propyl, n-Butyl, sec.-Butyl, tert.-Butyl, iso-Butyl, Pentyl, Hexyl, Heptyl, Octyl, Nonyl, Decyl, Dodecyl, Tetradecyl, Hexadecyl, Heptadecyl oder Octadecyl, wobei der Alkylrest substituiert sein kann, z.B. durch Hydroxy oder Alkoxy, und wobei die Alkylkette ein oder mehrfach unterbrochen sein kann, wie z.B. durch Sauerstoff, Schwefel, Amino-, Carbonamido-, Aminocarbonyl-, Ureido-, Sulfonamido-, Aminosulfonyl-, Carboxy-, und Carbonyloxy-.

Vorzugsweise ist A ein lineares oder verzweigtes C₁-C₁₈-Alkyl, insbesondere ein C₁-C₈-Alkyl, das mit OR₁ substituiert ist, wobei die Alkylkette mit einem oder mehreren Resten -Q- unterbrochen sein kann, oder ein lineares oder verzweigtes C₁-C₁₈-Alkyl, insbesondere ein C₁-C₈-Alkyl, wobei die Alkylkette mit einem oder mehreren Resten -Q- unterbrochen ist, worin -Q- -O-, -S-, -NR₂-, -CONR₂-, -NR₂CO-, -NR₂-CO-NR₃-, -SO₂NR₂-, -NR₂SO₂-, -COO-, -OCO-, -NR₂COO- oder -OCOO- ist,
R₁ Wasserstoff oder C₁-C₄-Alkyl ist,
R₂ Wasserstoff oder C₁-C₄-Alkyl ist und
R₃ Wasserstoff oder C₁-C₄-Alkyl ist.

C₁-C₁₈-Alkyl, bzw. C₁-C₈-Alkyl als A kann gegebenenfalls ein- oder mehrfach z.B. durch Carboxy, Carbonamido oder Sulfonamido weitersubstituiert sein.

Als X ist -NR- bevorzugt.

R in der Bedeutung von A ist vorzugsweise ein C₁-C₄-Alkyl, wie Methyl, Ethyl, n-Propyl, iso-Propyl, n-Butyl, sec.-Butyl, tert.-Butyl oder iso-Butyl.

Als R besonders bevorzugt ist Methyl und insbesondere Wasserstoff.

R₁ als C₁-C₄-Alkyl ist Methyl, Ethyl, n-Propyl, iso-Propyl, n-Butyl, sec.-Butyl, tert.-Butyl oder iso-Butyl.

R₂ und R₃ als C₁-C₄-Alkyl sind Methyl, Ethyl, n-Propyl, iso-Propyl, n-Butyl, sec.-Butyl, tert.-Butyl, oder iso-Butyl.

Als R₁ ist Methyl und insbesondere Wasserstoff bevorzugt.
Als R₂ ist Wasserstoff bevorzugt.
Als R₃ ist Wasserstoff bevorzugt.
Als -Q- ist -O-, -S- und -NH- bevorzugt.

Für das erfindungsgemässe Verfahren sind vom besonderen Interesse die Verbindungen der Formel (1), worin -X-A für folgende Reste steht:
-NHCH₂CH₂SCH₂CH₂OH, -NHCH₂CH₂CONHCH₂CH₂OH,
-NHCH₂CH₂CH₂CONHCH₂CH₂OH, -NHCH₂CONHCH₂CH₂CH₂OH,
-NHCH₂CONHCH₂CH₂CH₂OH, -NHCH₂CON(CH₂CH₂OH)₂,
-NHCH₂CH₂NHCOCH₂CH₂CH₂OH, -NHCH₂CH₂CH₂NHCOCH₂CH₂CH₂OH, und
-NHCH₂CH₂CH₂CONHCH₂CH₂CH₂OH.

Besonders wichtig für das erfindungsgemässe Verfahren sind die Verbindungen der Formeln 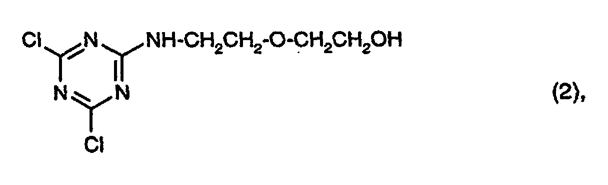 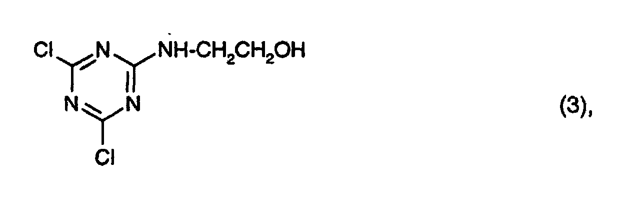 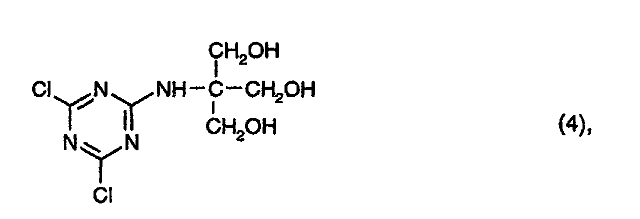 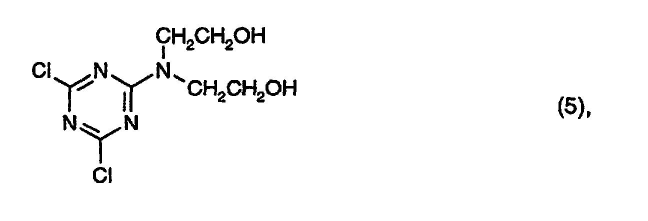 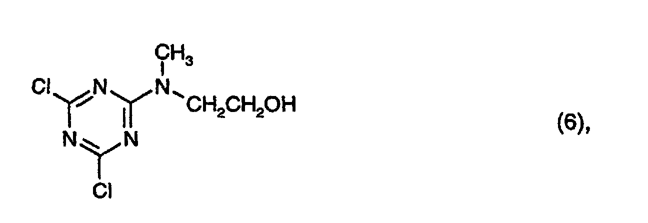 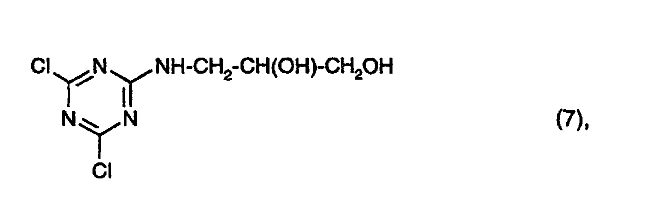 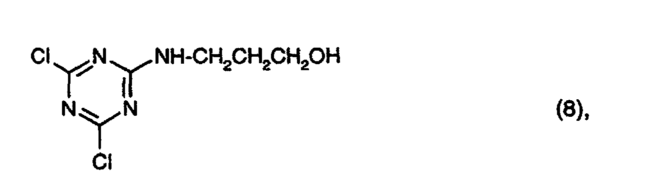 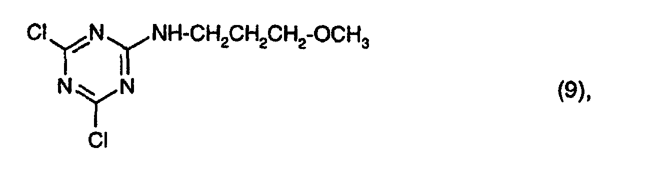 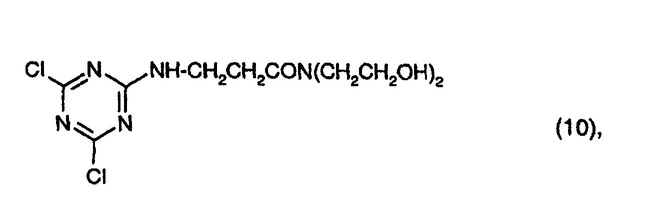 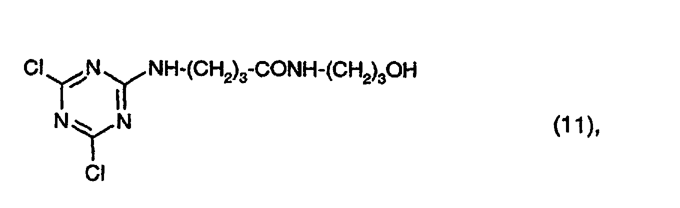 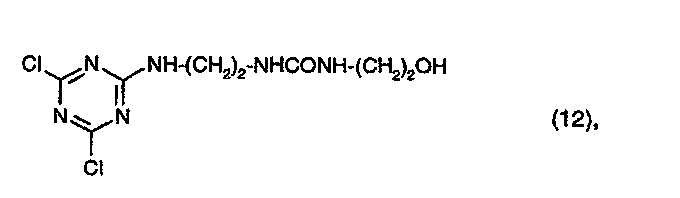 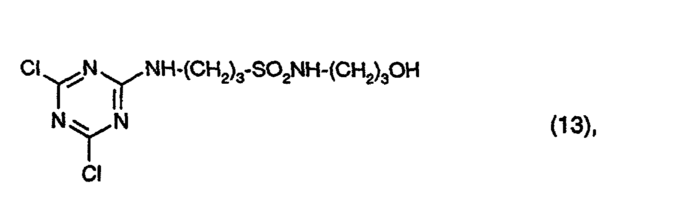 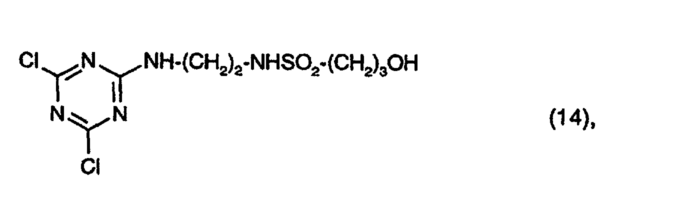 und

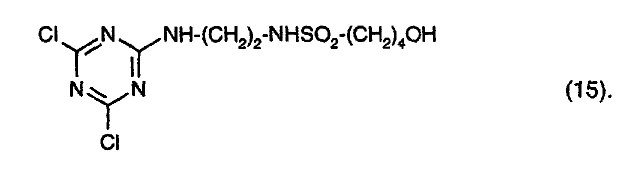

Ebenfalls besonders wichtig für das erfindungsgemässe Verfahren ist die Verbindung der Formel

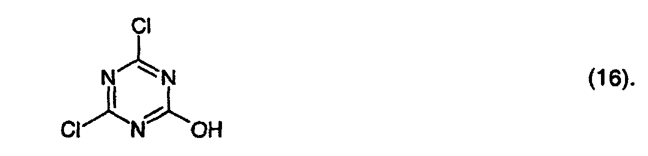

Ganz besonders wichtig für das erfindungsgemässe Verfahren sind die Verbindungen der Formeln (2) und (3).

Die Verbindungen der Formeln (2), (7) und (10) bis (15) sind neu und stellen einen weiteren Gegenstand der vorliegenden Erfindung dar.

Die Herstellung der in dem erfindungsgemässen Verfahren verwendeten Verbindungen der Formel (1), geschieht nach an sich bekannten Methoden, indem man z.B. ein Trichlortriazin der Formel 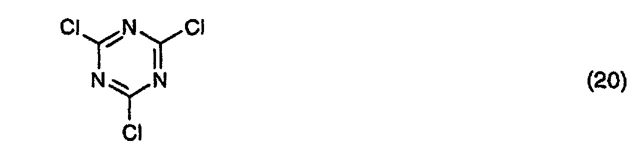 mit äquimolarer Menge einer Verbindung der Formel

HX-A (21),

worin X und A die unter der Formel (1) angegebene Bedeutung hat, umsetzt und das entstehende Endprodukt der Formel (1) isoliert.

Die Herstellung der neuen Verbindungen der Formeln (2), (7) und (10) bis (15) geschieht in analoger Weise, indem man ein Trichlortriazin der Formel (20) mit einem Amin der Formel

NH₂-CH₂CH₂OCH₂CH₂OH (22),

NH₂-CH₂CH(OH)CH₂OH (23),

NH₂-CH₂CH₂CON(CH₂CH₂OH)₂ (24),

NH₂-CH₂CH₂CH₂CONHCH₂CH₂CH₂OH (25),

NH₂-CH₂CH₂NHCONHCH₂CH₂OH (26),

NH₂-CH₂CH₂CH₂SO₂NHCH₂CH₂CH₂OH (27),

NH₂-CH₂CH₂NHSO₂CH₂CH₂CH₂OH (28)

oder

NH₂-CH₂CH₂NHSO₂CH₂CH₂CH₂CH₂OH (29)

umsetzt.
Die Amine der Formeln (22) bis (29) sind z.T. bekannt oder können nach an sich bekannten Methoden hergestellt werden.

Eine zweckmässige Ausführungsform des erfindungsgemässen Verfahrens ist dadurch gekennzeichnet, dass die Lyocell-Cellulosefasern mit der erfindungsgemäss verwendeten Verbindungen in einem alkalischen Milieu behandelt werden.
Das alkalische Milieu wird vorzugsweise von einem Alkylicarbonat und/oder einem Alkalihydroxid gebildet.
Die Behandlung kann dabei sowohl an ungefärbten Fasern, als auch an zu färbenden Fasern vor, während oder unmittelbar nach einem Färbeprozess, durchgeführt werden.

Werden die erfindungsgemäss verwendeten Verbindungen an ungefärbten Fasern appliziert, so kann es entweder mittels eines Behandlungsbades oder direkt nach dem Spinnvorgang an den frisch gesponnenen, noch nicht getrockneten Fasern (sog. "never dried"-Faser, wie sie z.B. in der EP-A-0 538 977 auf der Seite 4, oder in der US-A-5,580,354 in der Spalte 4 beschrieben sind) geschehen.

Werden die erfindungsgemäss verwendeten Verbindungen Im Laufe eines Färbeprozesses eingesetzt, so geschieht dies vorteilhafterweise vor oder während des eigentlichen Färbevorganges.
Die erfindungsgemäss verwendeten Verbindungen können dabei in einem separaten Behandlungsbad, oder vorzugsweise zusammen mit den verwendeten Farbstoffen in einem Färbebad bei den jeweiligen Färbebedingungen auf die Faser appliziert werden.
Werden die erfindungsgemäss verwendeten Verbindungen aus einem separaten Behandlungsbad auf die Faser gebracht, so kann dies bei einer Temperatur von 15 bis 140° C, vorzugsweise von 40 bis 100° C während 10 bis 120 Minuten, vorzugsweise 30 bis 90 Minuten, erfolgen. Eine weitere vorteilhafte Ausführung des erfindungsgemässen Verfahrens besteht darin, die zu behandelnden Fasern mit einer wässrigen Lösung der erfindungsgemäss verwendeten Verbindungen zu benetzen und anschliessend während 5 bis 60, vorzugsweise 10 bis 30 Sekunden einer Dampfbehandlung bei 90 bis 130° C unterziehen. Die Benetzung der Faser kann sowohl durch Eintauchen in ein Bad als auch durch Besprühen der Faser stattfinden.

Die Lyocell-Cellulosefasern können als solche, als Garn, oder in Form von Flächengebilden, wie z.B. Gewebe, Gestricke oder Gewirke vorliegen und behandelt werden.

Die Mengen in denen die erfindungsgemäss verwendeten Verbindungen in den Behandlungs- oder Färbeflotten eingesetzt werden liegen normalerweise zwischen 0,1 und 15 Gew.-%, insbesondere zwischen 1 und 10 Gew.-%, vor allem zwischen 2 und 6 Gew.-% bezogen auf das Gewicht der Faser.

Einen weiteren Gegenstand der vorliegenden Erfindung stellt die Verwendung der Verbindungen der Formel (1) zur Verringerung der Fibrillierung bei der Lyocell-Cellulose dar. Die Fibrillierungneigung der unbehandelten und behandelten Fasern wird nach einem modifiziertem Martindale Nassscheuertest, bei dem das genässte Gewebemuster bis zur ersten Lochbildung unter definierter Belastung gescheuert wird, beurteilt. Die Anzahl der Scheuertouren bis zur Lochbildung ergibt die Nassscheuerbeständigkeit, die als Massstab für die Fibrillierung dient.

Die nachfolgenden Beispiele dienen der Erläuterung der Erfindung. Die Temperaturen sind in Celsiusgraden angegeben, Teile sind Gewichtsteile, und Prozentangaben beziehen sich auf Gew.-%, sofern nicht anders vermerkt. Gewichtsteile stehen zu Volumenteilen im Verhältnis von Kilogramm zu Liter.

### Beispiel 1:

18,5 g Cyanurchlorid werden in eine Mischung bestehend aus 100 g fein gemahlenen Eis und 50 ml Wasser, welches 0,6 g eines handelsüblichen Tensids enthält, eingetragen und während 15 Minuten benetzt. Anschliessend werden bei guter Rührung 6,1 g Ethanolamin tropfenweise so zugesetzt, dass der pH-Wert der Mischung bei ca. 8 gehalten wird. Nach der beendeten Zugabe von Ethanolamin wird anschliessend ca. 8 g einer 50%-igen wässrigen Lösung von NaOH so zugetropft, dass der pH-Wert 8 gehalten werden kann. Die entstehende feine Suspension wird abfiltriert und getrocknet.
Man erhält 15,5 g einer weissen pulverigen Verbindung der Formel

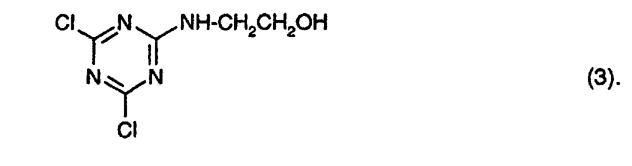

### Beispiele 2 bis 9:

Verfährt man wie im Beispiel 1 angegeben, verwendet aber anstatt 6,1 g Ethanolamin die äquivalente Menge eines der Amine der Formeln (22) bis (29), so erhält man die in der Tabelle 1 aufgeführten Verbindungen der Formeln (2), (7) und (10) bis (15).

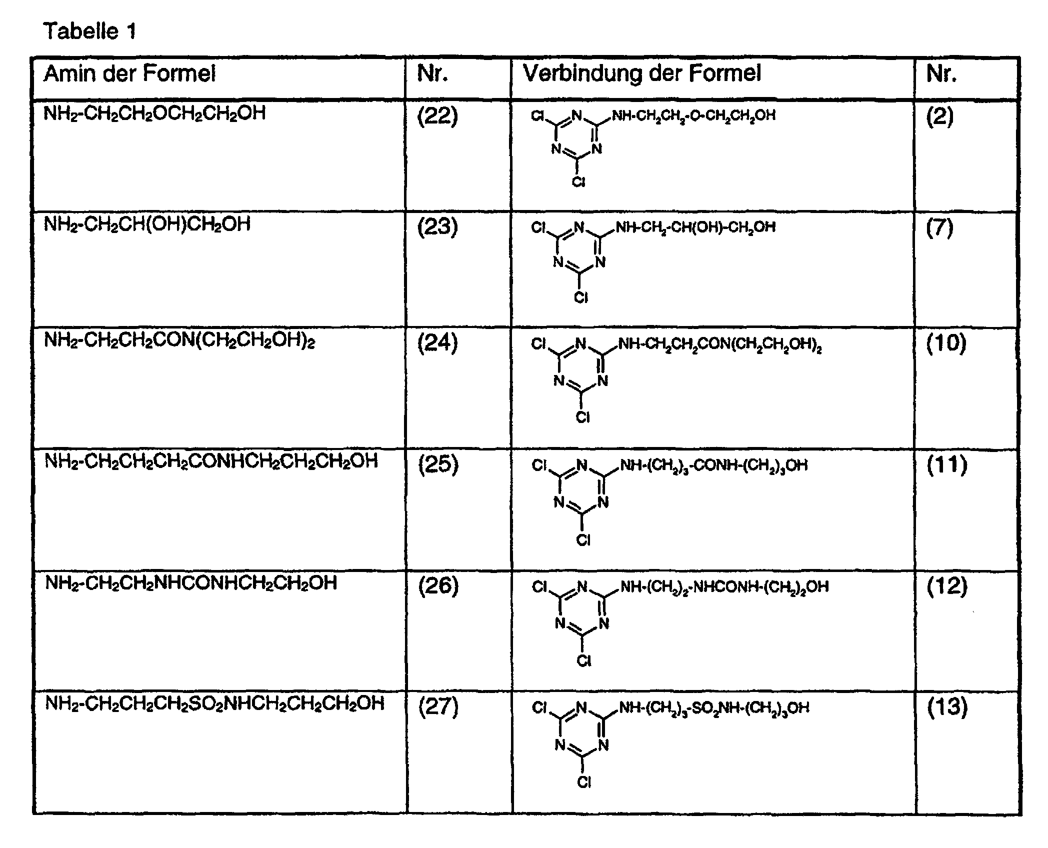

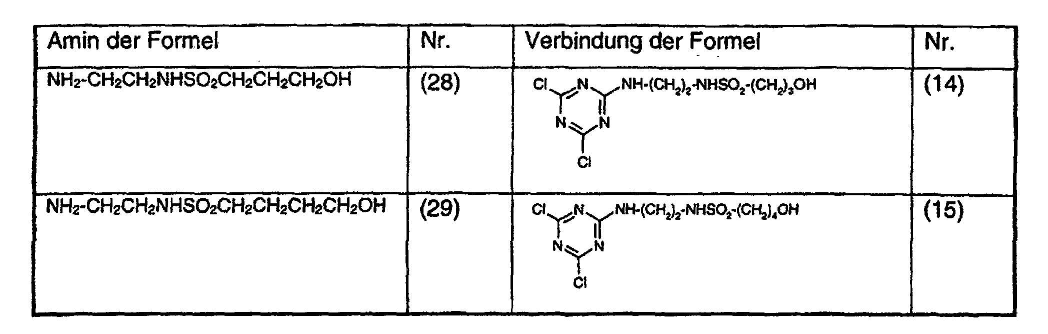

### Beispiel 10:

In einer Laborfärbeapparatur wird eine Flotte bestehend aus
50 ml Wasser und
15 ml einer 20%-igen wässrigen Lösung von Natriumsulfat
vorgelegt und auf 50° C aufgeheizt. Danach wird in die Flotte ein Stück von 10 g Lyocell-Gewebe eingetaucht und die Temperatur mit einem Gradient von 2° C/Min auf 90° C erhöht. Während der Aufheizphase werden bei 70° C 0,6 g der Verbindung der Formel (3), gelöst in 27,5 ml Wasser, zugegeben. Das Lyocell-Gewebe wird bei 90° C 60 Minuten be-handelt. Anschliessend wird die Behandlungsflotte auf 70° C abgekühlt, versetzt mit 7,5 ml einer 20%-igen wässrigen Soda-Lösung und weitere 45 Minuten bei 70° C gehalten.
Danach wird das Bad abgelassen und das behandelte Lyocell-Gewebe mit Wasser gespült, in frischem, nur aus Wasser bestehendem, Bad 5 Minuten gekocht, nochmals kalt gespült und getrocknet.
Das getrocknete Lyocell-Gewebe zeigt bei einem Martindale-Nassscheuertest eine ca. doppelte Anzahl Scheuertouren im Vergleich zu unbehandeltem Lyocell-Gewebe.
Bei dieser Applikation kann das Lyocell-Gewebe auch gleichzeitig gefärbt werden, indem ein oder mehrere Reaktivfarbstoffe direkt nach Zugabe der Verbindung der Formel (3) zugegeben werden.
Verfährt man wie im Beispiel 10 beschrieben, verwendet aber anstatt 0,6 g der Verbindung der Formel (3) die gleiche Menge einer der Verbindungen der Formeln (2) oder (4) bis (15), erhält man ebenfalls ein Lyocell-Gewebe mit hohen Martindale-Nassscheuertestwerten.

### Beispiel 11:

In einer Laborfärbeapparatur wird eine Flotte bestehend aus
50 ml Wasser und
15 ml einer 20%-igen wässrigen Lösung von Natriumsulfat
vorgelegt und auf 60° C aufgeheizt. Danach wird in die Flotte ein Stück von 10 g Lyocell-Gewebe eingetaucht und 3 Minuten später 0,6 g der Verbindung der Formel (3), gelöst in 25,5 ml Wasser zugegeben. Nach weiteren 15 Minuten wird die Flotte mit 7,5 ml einer 20%-igen wässrigen Soda-Lösung versetzt. Danach hält man die Flotte 30 Minuten bei 60° C.
Dann versetzt man die Flotte mit 2 ml einer 3%-igen wässrigen NaOH-Lösung, hält weitere 10 Minuten bei 60° C und stellt anschliessend das Lyocell-Gewebe wie in Beispiel 2 beschrieben fertig.
Das fertiggestellte Lyocell-Gewebe kann anschliessend nach üblichen Verfahren, mit z.B. Reaktivfarbstoffen, gefärbt werden.
Das getrocknete Lyocell-Gewebe zeigt bei einem Martindale-Nassscheuertest eine ca. doppelte Anzahl Scheuertouren im Vergleich zu unbehandeltem Lyocell-Gewebe.
Verfährt man wie im Beispiel 11 beschrieben, verwendet aber anstatt 0,6 g der Verbindung der Formel (3) die gleiche Menge einer der Verbindungen der Formeln (2) oder (4) bis (15), erhält man ebenfalls ein Lyocell-Gewebe mit hohen Martindale-Nassscheuertestwerten.

### Beispiel 12:

Eine Flotte enthaltend
42 g/l einer Verbindung der Formel 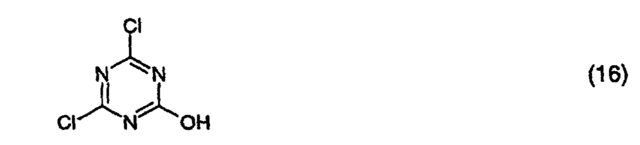 70 ml/l Wasserglas (38° Bé),
33 ml/l einer wässrigen Natriumhydroxydlösung (36° Bé), und
1 g/l eines handelsüblichen Netzmittels
wird auf ein Lyocell-Gewebe auffoulardiert (Flottenaufnahme 70%). Das so behandelte Lyocell-Gewebe wird ohne Trocknung direkt während 8 Minuten in Sattdampf von 102° C weiterbehandelt. Danach wird das Lyocell-Gewebe mit Wasser gespült, in frischem, rein wässrigen Bad 5 Minuten gekocht, kalt gespült und getrocknet.
Das behandelte Lyocell-Gewebe zeigt bei einem Martindale-Nassscheuertest im Vergleich zu unbehandeltem Lyocell-Gewebe eine ca. 2,5-fache Anzahl Scheuertouren.

### Beispiel 13:

In einer Laborfärbeapparatur wird eine Flotte bestehend aus
35 ml Wasser und
30 ml einer 20%-igen wässrigen Lösung von Natriumsulfat
vorgelegt und auf 80° C aufgeheizt. Danach wird in die Flotte ein Stück von 10 Lyocell-Gewebe eingetaucht und nach 5 Minuten
0,5 g der Verbindung der Formel (16) gelöst in 20 ml Wasser zugegeben. Nach weiteren 15 Minuten wird die Flotte mit 5 ml einer 20%-igen wässrigen Soda-Lösung versetzt. Nach weiteren 5 Minuten werden 5 ml einer 3%-igen wässrigen NaOH-Lösung zugesetzt. Nach weiteren 5 Minuten werden nochmals 5 ml einer 3%-igen wässrigen NaOH-Lösung zugesetzt. Anschliessend wird die Flotte 30 Minuten bei 80° C gehalten.
Danach wird das Bad abgelassen und das behandelte Lyocell-Gewebe mit Wasser gespült, in frischem, nur aus Wasser bestehendem Bad 5 Minuten gekocht, nochmals kalt gespült und getrocknet.
Das getrocknete Lyocell-Gewebe zeigt bei einem Martindale-Nassscheuertest eine mehr als doppelte Anzahl Scheuertouren im Vergleich zu unbehandeltem Lyocell-Gewebe.

## Patentansprüche

1. Verfahren zur Behandlung von Lyocell-Cellulosefasem, **dadurch gekennzeichnet, dass** man die Lyocell-Cellulosefaser mit mindestens einer Verbindung der Formel 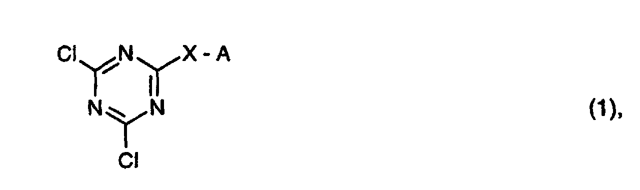 worin
X -NR- oder -S- ist, A einen aliphatischer Rest mit 1 bis 18 C-Atomen, der frei von Sulfogruppen ist, bedeutet, oder -X-A Hydroxy ist, und R Wasserstoff ist oder die Bedeutung von A unabhängig von A hat,
behandelt.

2. Verfahren gemäss Anspruch 1, **dadurch gekennzeichnet, dass** man die Lyocell-Cellulosefaser mit mindestens einer Verbindung der Formel (1), worin X -NR- oder -S- ist, A einen aliphatischer Rest mit 1 bis 18 C-Atomen, der frei von Sulfogruppen ist, bedeutet, und R Wasserstoff ist oder die Bedeutung von A unabhängig von A hat,
behandelt.

3. Verfahren gemäss Anspruch 1, **dadurch gekennzeichnet, dass** man die Lyocell-Cellulosefaser mit mindestens einer Verbindung der Formel 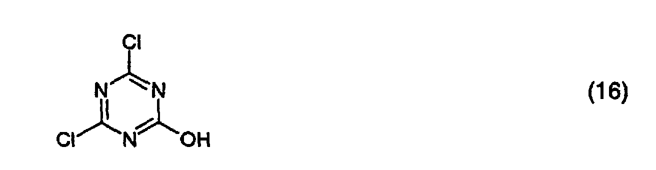 behandelt.

4. Verfahren gemäss einem der Ansprüche 1 und 2, **dadurch gekennzeichnet, dass** A ein C₁-C₁₈-Alkyl, das mit OR₁ substituiert ist, wobei die Alkylkette mit einem oder mehreren Resten -Q- unterbrochen sein kann, oder ein C₁-C₁₈-Alkyl, wobei die Alkylkette mit einem oder mehreren Resten -Q- unterbrochen ist, worin -Q- -O-, -S-, -NR₂-, -CONR₂-, -NR₂CO-, -NR₂-CO-NR₃-, -SO₂NR₂-, -NR₂SO₂-, -COO-, -OCO-, -NR₂COO- oder -OCOO- ist und R₁ Wasserstoff oder C₁-C₄-Alkyl, R₂ Wasserstoff oder C₁-C₄-Alkyl und R₃ Wasserstoff oder C₁-C₄-Alkyl bedeuten.

5. Verfahren gemäss Anspruch 4, **dadurch gekennzeichnet, dass** A ein C₁-C₈-Alkyl, das mit OR₁ substituiert ist, wobei die Alkylkette mit einem oder mehreren Resten -Q- unterbrochen sein kann, oder ein C₁-C₈-Alkyl, wobei die Alkylkette mit einem oder mehreren Resten -Qunterbrochen ist, worin -Q- -O-, -S-, -NR₂-, -CONR₂-, -NR₂CO-, -NR₂-CO-NR₃-, -SO₂NR₂-, -NR₂SO₂-, -COO-, -OCO-, -NR₂COO- oder -OCOO- ist und R₁ Wasserstoff oder C₁-C₄-Alkyl, R₂ Wasserstoff oder C₁-C₄-Alkyl und R₃ Wasserstoff oder C₁-C₄-Alkyl bedeuten.

6. Verfahren gemäss einem der Ansprüche 1, 2, 4 und 5 **dadurch gekennzeichnet, dass** X -NR- ist.

7. Verfahren gemäss einem der Ansprüche 4 bis 6, **dadurch gekennzeichnet, dass** -Q- -O-, -S- oder -NR₂- ist.

8. Verfahren gemäss Anspruch 1, **dadurch gekennzeichnet, dass** man eine der Verbindungen der Formeln 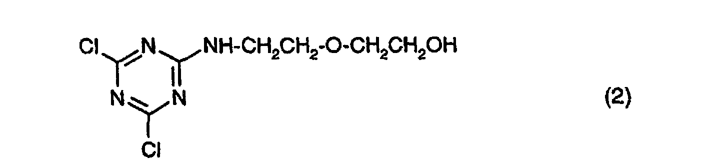 und 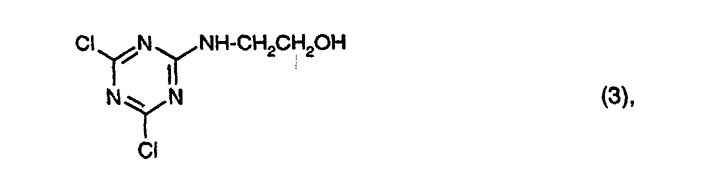 verwendet.

9. Verfahren gemäss einem der Ansprüche 1, 2 und 4 bis 7, **dadurch gekennzeichnet, dass** man zwischen 0,1 und 15 Gew.-% der Verbindung der Formel (1), bezogen auf das Gewicht der Faser, verwendet.

10. Verfahren gemäss Anspruch 3, **dadurch gekennzeichnet, dass** man zwischen 0,1 und 15 Gew.-% der Verbindung der Formel (16), bezogen auf das Gewicht der Faser, verwendet.

11. Verfahren gemäss Anspruch 8, **dadurch gekennzeichnet, dass** man zwischen 0,1 und 15 Gew.-% einer der Verbindungen der Formel (2) oder (3), bezogen auf das Gewicht der Faser, verwendet.

12. Verfahren gemäss einem der Ansprüche 1, 2, 4 bis 7 und 9, **dadurch gekennzeichnet, dass** man die Verbindung der Formel (1) an die frisch gesponnenen, noch nicht getrockneten Faser, appliziert.

13. Verfahren gemäss Anspruch 3, **dadurch gekennzeichnet, dass** man die Verbindung der Formel (16) an die frisch gesponnenen, noch nicht getrockneten Faser, appliziert.

14. Verfahren gemäss Anspruch 8, **dadurch gekennzeichnet, dass** man eine der Verbindungen der Formel (2) oder (3) an die frisch gesponnenen, noch nicht getrockneten Faser, appliziert.

15. Verfahren gemäss Anspruch 1, **dadurch gekennzeichnet, dass** man die Verbindung der Formel (1) vor oder während eines Färbeprozesses auf die Faser appliziert.

16. Verbindungen gemäss Anspruch 1 der Formel (1), worin -X-A -NH(CH₂)₂O(CH₂)₂OH, -NHCH₂CH(OH)CH₂OH, -NH(CH₂)₂CON(CH₂CH₂OH)₂, -NH(CH₂)₃CONH(CH₂)₃OH, -NH(CH₂)₂NHCONH(CH₂)₂OH, -NH(CH₂)₃SO₂NH(CH₂)₃OH, -NH(CH₂)₂NHSO₂(CH₂)₃OH, oder -NH(CH₂)₂NHSO₂(CH₂)₄OH bedeutet.

17. Verbindungen gemäss Anspruch 16 der Formel 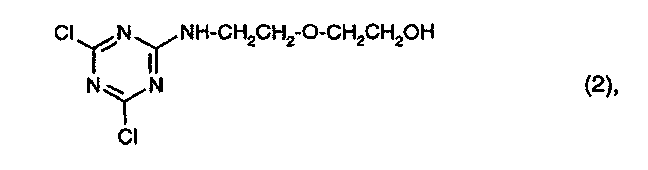 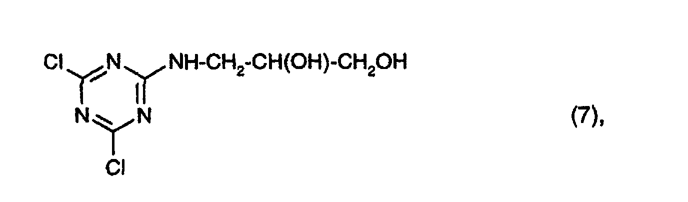 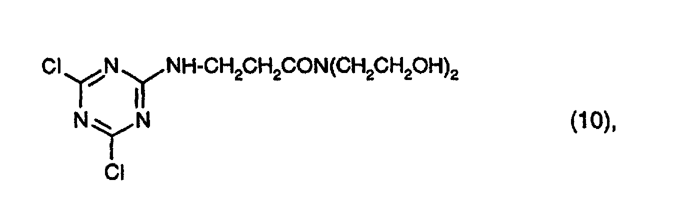 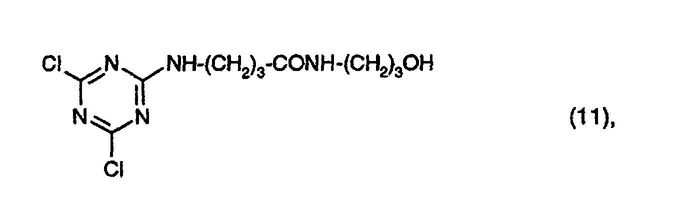 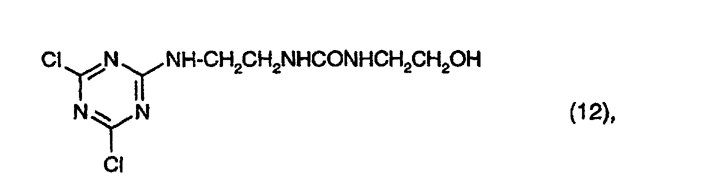 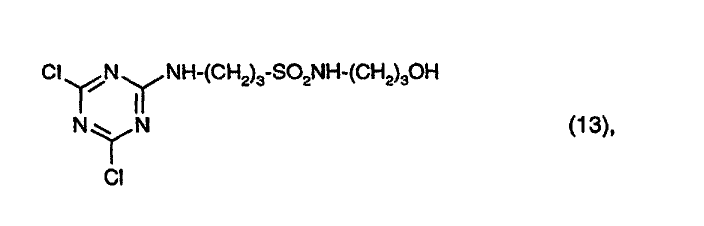 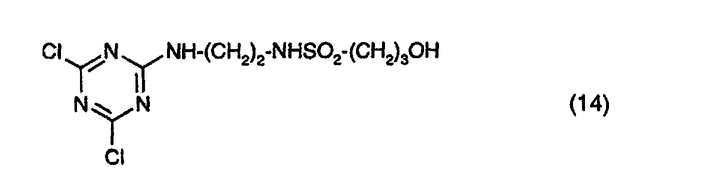 und

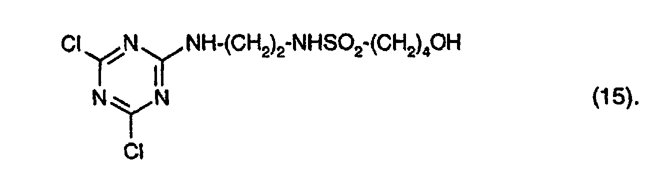

18. Verwendung der Vebindungen gemäss Anspruch 1 zur Verringerung der Fibrillierung bei Lyocell-Cellulosefasern.

## Claims

1. A process for the treatment of Lyocell cellulose fibres, **characterized in that** the Lyocell cellulose fibre is treated with at least one compound of the formula 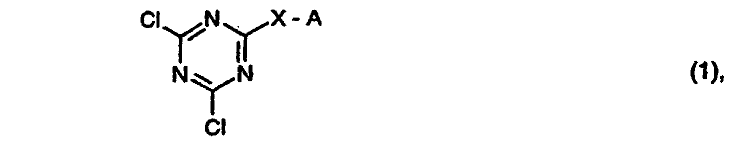 wherein X is -NR- or -S-, A represents an aliphatic residue comprising 1 to 18 C-atoms and being free from sulfonic groups, or -X-A is hydroxy, and R is hydrogen or has the meaning of A irrespective of A.

2. A process according to claim 1, **characterized in that** the Lyocell cellulose fibre is treated with at least one compound of formula (1), wherein X is -NR- or -S-, A represents an aliphatic residue comprising 1 to 18 C-atoms and being free from sulfonic groups, and R is hydrogen or has the meaning of A irrespective of A.

3. A process according to claim 1, **characterized in that** the Lyocell cellulose fibre is treated with at least one compound of the formula:

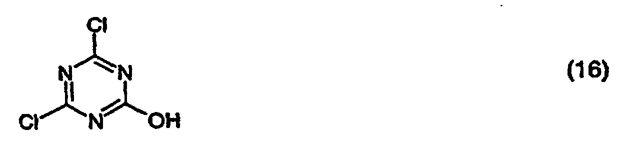

4. A process according to any one of claims 1 and 2, **characterized in that** A is a C₁-C₁₈-alkyl which is substituted with OR₁, wherein the alkyl chain may be interrupted with one or several residues -Q-, or a C₁-C₁₈-alkyl, wherein the alkyl chain is interrupted with one or several residues -Q-, wherein -Q- is -O-, -S-, -NR₂-, -CONR₂-, -NR₂CO-, -NR₂-CO-NR₃-, -SO₂NR₂-, -NR₂SO₂-, -COO-, -OCO-, -NR₂COO- or -OCOO- and R₁ represents hydrogen or C₁-C₄-alkyl, R₂ represents hydrogen or C₁-C₄-alkyl and R₃ represents hydrogen or C₁-C₄-alkyl.

5. A process according to claim 4, **characterized in that** A is a C₁-C₈-alkyl which is substituted with OR₁, wherein the alkyl chain may be interrupted with one or several residues -Q-, or a C₁-C₈-alkyl, wherein the alkyl chain is interrupted with one or several residues -Q-, wherein -Q- is -O-, -S-, -NR₂-, -CONR₂-, -NR₂CO-, -NR₂-CO-NR₃-, -SO₂NR₂-, -NR₂SO₂-, -COO-, -OCO-, -NR₂COO- or -OCOO- and R₁ represents hydrogen or C₁-C₄-alkyl, R₂ represents hydrogen or C₁-C₄-alkyl and R₃ represents hydrogen or C₁-C₄-alkyl.

6. A process according to any one of claims 1, 2, 4 and 5, **characterized in that** X is -NR-.

7. A process according to any one of claims 4 to 6, **characterized in that** -Q- is -O-, -S- or -NR₂-.

8. A process according to claim 1, **characterized in that** one of the compounds of the formula 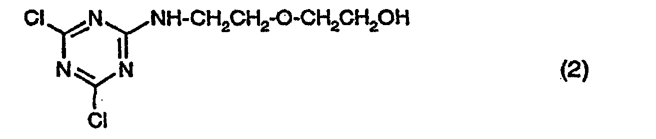 and 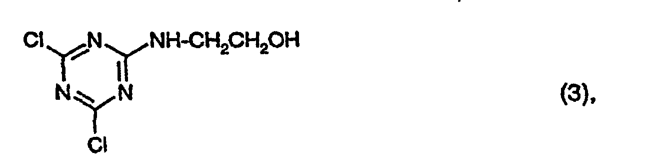 is used.

9. A process according to any one of claims 1, 2 and 4 to 7, **characterized in that** between 0.1 and 15 % by weight of the compound of formula (1), based on the weight of the fibre, is used.

10. A process according to claim 3, **characterized in that** between 0.1 and 15 % by weight of the compound of formula (16), based on the weight of the fibre, is used.

11. A process according to claim 8, **characterized in that** between 0.1 and 15 % by weight of one of the compounds of formula (2) or (3), based on the weight of the fibre, is used.

12. A process according to any one of claims 1, 2, 4 to 7 and 9, **characterized in that** the compound of formula (1) is applied onto the freshly spun, not yet dried fibres.

13. A process according to claim 3, **characterized in that** the compound of formula (16) is applied onto the freshly spun, not yet dried fibres.

14. A process according to claim 8, **characterized in that** one of the compounds of formula (2) or (3) is applied onto the freshly spun, not yet dried fibres.

15. A process according to claim 1, **characterized in that** the compound of formula (1) is applied onto the fibre prior to or during a dyeing process.

16. Compounds according to claim 1 of formula (1), wherein -X-A represents
-NH(CH₂)₂O(CH₂)₂OH, -NHCH₂CH(OH)CH₂OH, -NH(CH₂)₂CON(CH₂CH₂OH)₂,
-NH(CH₂)₃CONH(CH₂)₃OH, -NH(CH₂)₂NHCONH(CH₂)₂OH,
-NH(CH₂)₃SO₂NH(CH₂)₃OH, -NH(CH₂)₂NHSO₂(CH₂)₃OH or
-NH(CH₂)₂NHSO₂(CH₂)₄OH.

17. Compounds according to claim 16 of the formula 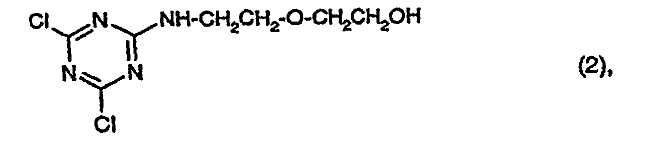 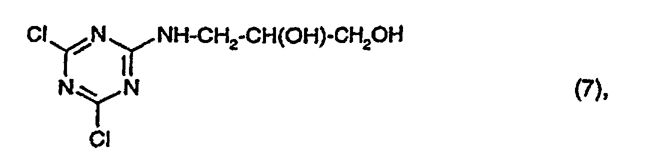 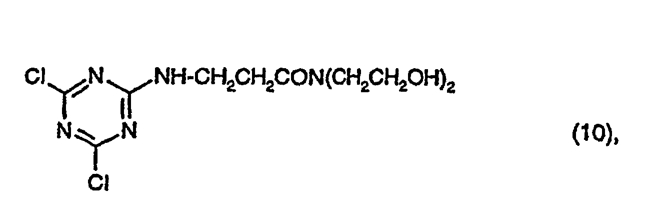 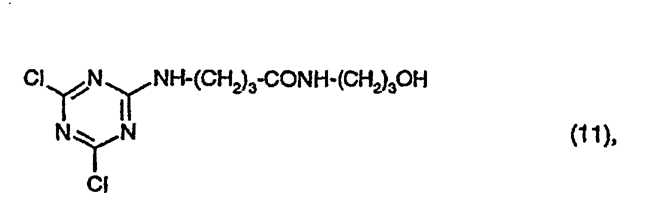 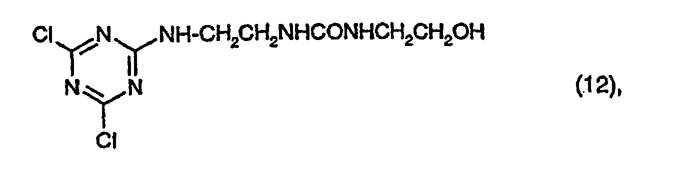 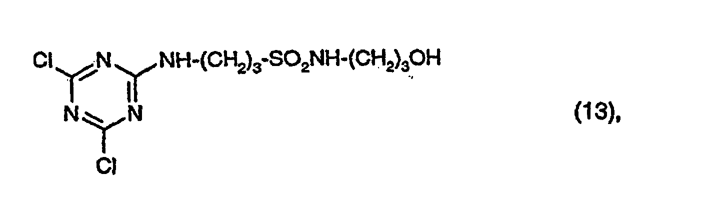 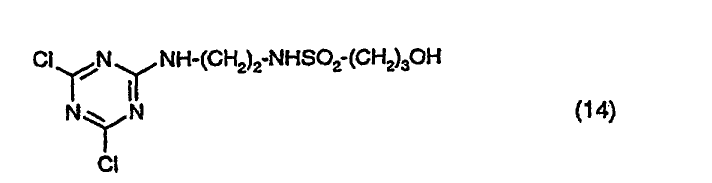 and

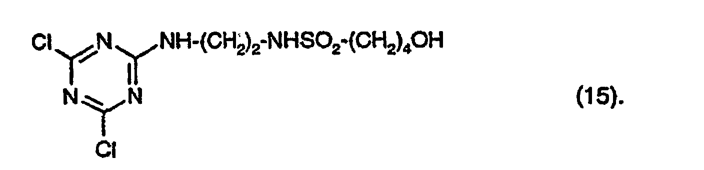

18. The use of the compounds according to claim 1 for reducing the fibrillation of Lyocell cellulose fibres.

## Revendications

1. Procédé pour le traitement de fibres de cellulose Lyocell, **caractérisé en ce que** l'on traite la fibre de cellulose Lyocell avec au moins un composé de formule 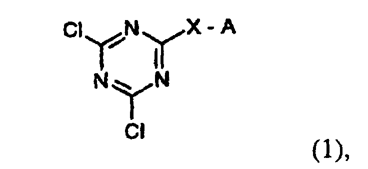 où X représente -NR- ou -S-, A est un reste aliphatique ayant 1 à 18 atomes de carbone ne contenant pas de groupe sulfo, ou -X-A est un hydroxyle et R est un hydrogène ou a la même définition que A, indépendamment de A.

2. Procédé selon la revendication 1, **caractérisé en ce que** l'on traite la fibre de cellulose Lyocell avec au moins un composé de formule (1) où X représente -NR- ou -S-, A est un reste aliphatique ayant 1 à 18 atomes de carbone ne contenant pas de groupe sulfo et R est un hydrogène ou a la même définition que A, indépendamment de A.

3. Procédé selon la revendication 1, **caractérisé en ce que** l'on traite la fibre de cellulose Lyocell avec au moins un composé de formule

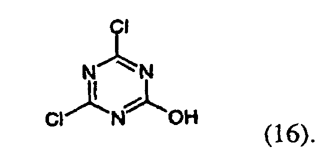

4. Procédé selon l'une des revendications 1 et 2, **caractérisé en ce que** A est un reste alkyle en C₁-C₁₈, substitué par OR₁, la chaîne alkyle pouvant être interrompue par un ou plusieurs restes -Q-, ou un reste alkyle en C₁-C₁₈, la chaîne alkyle étant interrompue par un ou plusieurs restes -Q-, où -Q- est -O-, -S-, -NR₂-, -CONR₂-, -NR₂CO-, -NR₂-CO-NR₃-, -SO₂NR₂-, -NR₂SO₂-, -COO-, -OCO-, -NR₂COO- ou -OCOO- et R₁ représente un hydrogène ou un reste alkyle en C₁-C₄, R₂ un hydrogène ou un reste alkyle en C₁-C₄ et R₃ un hydrogène ou un reste alkyle en C₁-C₄.

5. Procédé selon la revendication 4, **caractérisé en ce que** A est un reste alkyle en C₁-C₈, substitué par OR₁, la chaîne alkyle pouvant être interrompue par un ou plusieurs restes -Q-, ou un reste alkyle en C₁-C₈, la chaîne alkyle étant interrompue par un ou plusieurs restes -Q-, où -Q- est -O-, -S-, -NR₂-, -CONR₂-, -NR₂CO-, -NR₂-CO-NR₃-, -SO₂NR₂-, -NR₂SO₂-, -COO-, -OCO-, -NR₂COO- ou -OCOO- et R₁ représente un hydrogène ou un reste alkyle en C₁-C₄, R₂ un hydrogène ou un reste alkyle en C₁-C₄ et R₃ un hydrogène ou un reste alkyle en C₁-C₄.

6. Procédé selon l'une quelconque des revendications 1, 2, 4 et 5, **caractérisé en ce que** X est -NR-.

7. Procédé selon l'une quelconque des revendications 4 à 6, **caractérisé en ce que** -Q- est -O-, -S- ou -NR₂-.

8. Procédé selon la revendication 1, **caractérisé en ce que** l'on utilise un des composés de formule 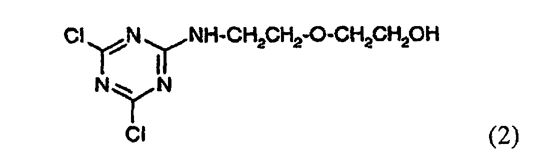 et

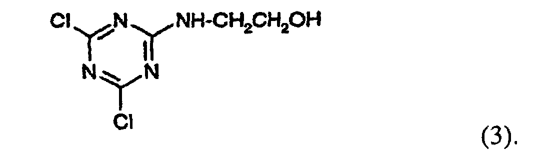

9. Procédé selon l'une quelconque des revendications 1, 2 et 4 à 7, **caractérisé en ce que** l'on utilise entre 0,1 et 15 % en masse de composé de formule (1) par rapport à la masse de la fibre.

10. Procédé selon la revendication 3, **caractérisé en ce que** l'on utilise entre 0,1 et 15 % en masse du composé de formule (16) par rapport à la masse de la fibre.

11. Procédé selon la revendication 8, **caractérisé en ce que** l'on utilise entre 0,1 et 15 % en masse d'un des composés de formule (2) ou (3) par rapport à la masse de la fibre.

12. Procédé selon l'une quelconque des revendications 1, 2, 4 à 7 et 9, **caractérisé en ce que** l'on applique le composé de formule (1) sur la fibre fraîchement filée, non-encore séchée.

13. Procédé selon la revendication 3, **caractérisé en ce que** l'on applique le composé de formule (16) sur la fibre fraîchement filée, non-encore séchée.

14. Procédé selon la revendication 8, **caractérisé en ce que** l'on applique l'un des composés de formule (2) ou (3) sur la fibre fraîchement filée, non-encore séchée.

15. Procédé selon la revendication 1, **caractérisé en ce que** l'on applique le composé de formule (1) sur la fibre avant ou pendant un procédé de coloration.

16. Composés de formule (1) selon la revendication 1, où -X-A
représente -NH(CH₂)₂O(CH₂)₂OH, -NHCH₂CH(OH)CH₂OH,
-NH(CH₂)₂CON(CH₂CH₂OH)₂, -NH(CH₂)₃CONH(CH₂)₃OH,
-NH(CH₂)₂NHCONH(CH₂)₂OH, -NH(CH₂)₃SO₂NH(CH₂)₃OH,
-NH(CH₂)₂NHSO₂(CH₂)₃OH ou -NH(CH₂)₂NHSO₂(CH₂)₄OH.

17. Composés selon la revendication 16 de formule 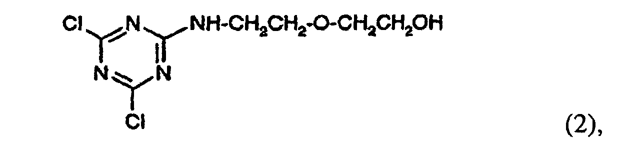 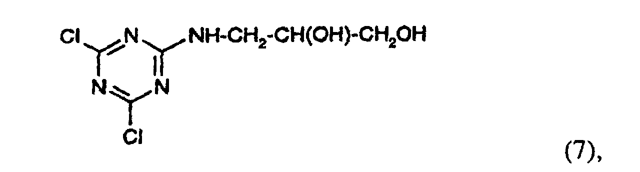 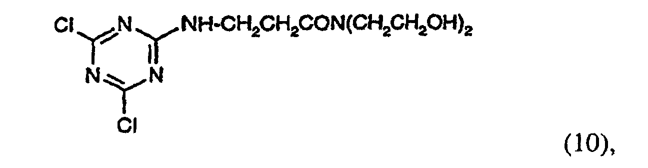 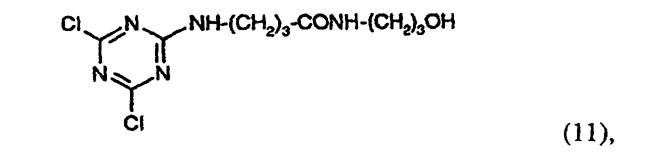 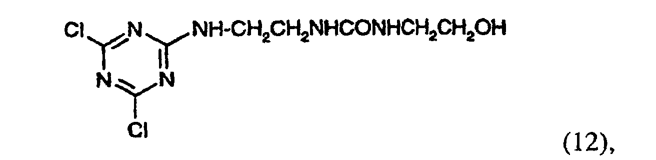 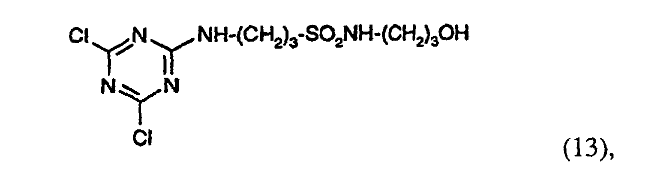 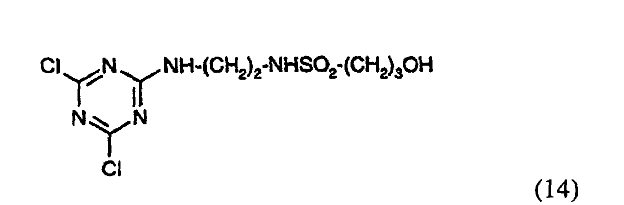 et

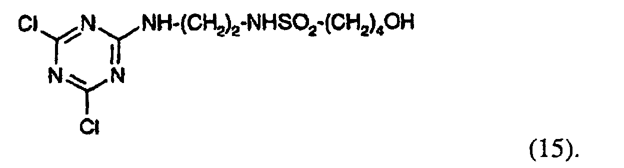

18. Utilisation des composés selon la revendication 1 pour diminuer la fibrillation des fibres de cellulose Lyocell.
